# EUROPEAN PATENT SPECIFICATION

(11) **EP 3 882 613 B1**
(45) Date of publication and mention of the grant of the patent: **31.12.2025**
(21) Application number: 20164217.0
(22) Date of filing: 19.03.2020
(51) Int. Cl.: G01N 27/12, G01L 1/18, G01L 1/22

(54) **STIMULI-RESPONSIVE SENSOR SYSTEM, DIGITAL LOGIC ELEMENT, ROBOTIC SYSTEM AND METHOD FOR DETECTING AN EXTERNAL STIMULUS**
AUF STIMULI REAGIERENDES SENSORSYSTEM, DIGITALES LOGIKELEMENT, ROBOTERSYSTEM UND VERFAHREN ZUR ERKENNUNG EXTERNER STIMULI
SYSTÈME DE CAPTEUR RÉAGISSANT AUX STIMULI, ÉLÉMENT LOGIQUE NUMÉRIQUE, SYSTÈME ROBOTIQUE ET PROCÉDÉ PERMETTANT DE DÉTECTER UN STIMULUS EXTERNE

(43) Date of publication of application: 22.09.2021
(73) Proprietor: Technische Universität Dresden, 01069 Dresden (DE)
(72) Inventor: Henke, Ernst-Friedrich Markus, 01099 Dresden (DE); Wilson, Ketherine Elizabeth, 1010 Auckland (NZ); Anderson, Iain Alexander, 1010 Auckland (NZ); Green, Ross, 1010 Auckland (NZ); Richter, Andreas, 01219 Dresden (DE); Franke, Markus, 01277 Dresden (DE); Gerlach, Gerald, 01069 Dresden (DE)
(74) Representative: Pfenning, Meinig & Partner mbB

(56) References cited:
- WO-A1-2018/091317
- CN-Y- 201 051 025
- US-A- 5 512 882
- US-A1- 2018 018 055
- US-B1- 10 107 827
- HENKE E-F MARKUS ET AL: "Entirely soft dielectric elastomer robots", PROCEEDINGS OF SPIE; [PROCEEDINGS OF SPIE ISSN 0277-786X VOLUME 10524], SPIE, US, vol. 10163, 17 April 2017 (2017-04-17), pages 101631N - 101631N, XP060090177, ISBN: 978-1-5106-1533-5, DOI: 10.1117/12.2260361

## Description

The present invention relates to a stimuli-responsive sensor system, a digital logic element and a robotic system comprising a stimuli-responsive sensor system or a digital logic element. The invention further relates to a method of detecting an external stimulus by a stimuli-responsive sensor system or a digital logic element.

Currently most sensor systems are rigid and complex. They comprise a multitude of different materials. These materials are typically stiff and inflexible, such as silicon. Therefore, even today only few soft sensor systems are available for soft robotics applications despite the high demand for entirely soft, compliable sensor system, e. g. for robotic sensory organs in the field of medical or surgical assistance.

In the prior art, sensor systems with soft polymer measuring elements are known. The sensor systems disclosed in DE 19828093 C2, for example, employ soft stimuli-responsive polymers as measuring elements. The sensor systems, however, rely on rigid and bulky transducers or indicating devices, which renders them unsuitable for soft robotics applications. US 10 107 827 B1 discloses a biomedical sensor utilizing a polymeric material. E.-F. Markus Henke et al.: "Entirely soft dielectric elastomer robots", Proc. of SPIE Vol. 10163, 101631N, deals with multifunctional dielectric elastomers used for robotic structures. An array piezoelectric sensing device is known from CN 201 051 025 Y. US 5 512 882 A discloses an apparatus for vapor detection with a polymer-based sensor.

The present invention is therefore based on the object to overcome these disadvantages providing a stimuli-responsive sensor system, a digital logic element, a robotic system comprising a stimuli-responsive sensor system or a digital logic element and a method for determining an external stimulus by a stimuli-responsive sensor system or a digital logic element.

This object is achieved according to the invention by a stimuli-responsive sensor system as claimed in claim 1, a digital logic element as claimed in claim 6, a robotic system as claimed in claim 7 and a method for determining an external stimulus as claimed in claim 8. Advantageous developments and embodiments are described in the dependent claims.

The sensory system enables a highly sensitive evaluation of external stimuli without the need of conventional rigid electronics. Since the system can be built entirely from soft components it constitutes a soft sensor system, which can be integrated in soft robotic structures, especially in biologically inspired or miniaturized soft structures. The use of soft sensor systems in robotics not only raises the acceptance by users but also reduces the risk of injuries during interaction of a robotic structure with the user.

In this application any material is meant to be regarded as a soft material, elastomeric material or elastomer if the Young's-modulus of the material is within a range of 1 x 10⁴ MPa to 1 x 10⁹ MPa, and as a rigid or stiff material if the Young's-modulus of the material is larger than 1 x 10⁹ MPa. The Young's modulus represents the linear relationship between the stress or force per unit surface and the strain or proportional deformation of the material when a load is applied to the material in uniaxial compression or extension. A soft system or soft structure is a system or structure which behaves in its entity like a soft material.

Stimuli-responsive elastomers are elastomers which can undergo large reversible changes in their physical properties in response to small external stimuli. In particular, a reversible change in a volume of the respective elastomer in response to a small external stimulus is to be covered by the term "stimuli-responsive elastomer". Preferably, the change in the volume is a change of at least 10 percent of the initial volume. Typically, the external stimuli comprises changes in chemical properties of an environment like a change in the pH-value or a change in a concentration of glucose. The term "small" in this regard is meant to cover changes of no more than 5 percent of the initial value. Due to their ability to respond to even small changes in their surrounding environment, they are also termed environmentally sensitive elastomers, smart elastomers or intelligent elastomers.

Dielectric elastomer switches can generally be piezoresistive elastomeric composites which exhibit a reversible change in their electric resistivity when the piezoresistive elastomeric composite is compressed or stretched. In the present sensor system compression or extension of the dielectric elastomer switch is achieved through the pressure which is applied by the stimuli-responsive elastomer on the contact surface between the dielectric elastomer switch and the stimuli-responsive elastomer.

Depending on the composition of the piezoresistive elastomeric composite a change in its electric resistivity can arise from different effects. Piezoresistive elastomeric composites can, for example, comprise a strain gauge made of an electrically conducting metal or a piezoresistive semiconductor, meaning a piezoresistor, which is integrated or embedded in an electrically insulating elastomeric matrix. If the strain gauge is designed with a high ratio of length to cross-sectional area, as for example in foil strain gauges, it can be flexible to a certain extent and the composite can thus behave like an elastomer despite its semiconductive or metallic components. In these piezoresistive elastomeric composites the change in resistivity results from the piezoresistive effect of the semiconductor material or from the change in the length and cross-sectional area of the electrically conducting metal in the strain gauge.

Alternatively, piezoresistive elastomeric composites can utilize percolation and/or quantum tunnelling effects. Such piezoresistive elastomeric composites consist of an electrically conducting filler that is finely dispersed in an electrically insulating elastomeric matrix. The filler may be coated with a polymer coating that constitutes a tunnelling barrier for charge carriers. If the composite is compressed or stretched, the electrically conducting filler is spatially rearranged and thus the conducting pathways in the piezoresistive elastomeric composite are altered and/or the tunnelling barriers throughout the filler are altered resulting in a change in the resistivity of the piezoresistive elastomeric composite.

In the present sensor system, the dielectric elastomer switch is preferably constructed with a piezoresistive elastomeric composite structure comprising an electrically conducting filler that is dispersed in a dielectric elastomeric matrix. The piezoresistive elastomeric composite structure itself may be embedded in an electrically insulating elastomer and is configured to exhibit an elastic deformation that changes the electrical resistivity of the piezoresistive elastomeric composite structure when a pressure is exerted on the piezoresistive elastomeric composite structure by the stimuli-responsive elastomer. The change in the resistivity can be detected or used to generate an electric output signal. In this application, any material is meant to be regarded as an electrically conducting material which exhibits an electrical conductivity of more than or equal to 10⁻⁸ S·m⁻¹ at room temperature, i. e. 20 °C, and an electrically insulating material which exhibits an electrical conductivity of less than 10⁻⁸ S·m⁻¹ at room temperature. Electrically insulating materials which can be polarized by an externally applied electric field are regarded as dielectric materials.

The electrically conducting filler of the piezoelectric elastomeric composite structure preferably comprises electrically conducting particles with a low aspect ratio which lead to a decrease of the resistivity of the piezoelectric elastomeric composite structure under compression or expansion of the piezoelectric elastomeric composite. Such particles may, for example, consist of or comprise nickel, silver or carbon, and may be of an average size between 10 nm and 10 µm.

The dielectric elastomeric matrix in which the electrically conducting filler is dispersed may be an elastomer or a viscoelastic fluid or foam. The concentration of the particles in the dielectric elastomeric matrix can be chosen below the percolation threshold of the piezoelectric elastomeric composite structure so that a prompt insulator-to-conductor transition occurs when the dielectric elastomer switch is a compressed, or above the percolation threshold so that a prompt conductor-to-insulator transition occurs when the dielectric elastomer switch is a stretched. The percolation threshold is the critical volume concentration of the filler at which the electric property of the piezoelectric elastomeric composite changes from the conductivity of the dielectric elastomeric matrix to the conductivity of the electrically conducting filler.

The piezoelectric elastomeric composite structure may be placed in direct contact to the stimuli responsive elastomer. However, by embedding the piezoelectric elastomeric composite structure in an electrically insulating elastomer, the pressure exerted by the stimuli responsive elastomer can be applied more uniformly to the piezoelectric elastomeric composite structure and can be used to insulate the dielectric elastomer switch from conductive fluids that interact with the stimuli-responsive elastomer. Furthermore, the electrically insulating elastomer may provide a support to delicate piezoelectric elastomeric composite structures and a protection against external overpressure, vibrations and also damaging external stimuli. As a result, a robust and easy to handle sensor system with a high reproducibility of the measurements, a high signal-to-noise ratio and a long life time can be provided.

The shape of the piezoresistive elastomeric composite structure can be two-dimensional, i. e. planar, or three-dimensional. The piezoresistive elastomeric composite structure typically comprises multiple elongated sections connected in series which are compressed or stretched when a pressure is applied to the dielectric elastomer switch. The elongated sections may, for example, be formed by wire-like tracks or pipes which are configured in the shape of a square, rectangular, triangular and/or meander-like wave.

For a high sensitivity of the dielectric elastomer switch the elongated sections are preferably oriented with their elongated axis parallel to the direction of the pressure action, i. e. perpendicular to the contact surface between the dielectric elastomer switch and the stimuli-responsive elastomer. The change in resistivity may thus be achieved not only by a percolation effect of the electrically conducting filler in the piezoresistive elastomeric composite structure but also by a change of the geometry, i. e. by a change of the cross-sectional-area and the length of the piezoresistive elastomeric composite structure. Consequently, a soft sensor system with a high dynamic range of sensitivity can be provided. As a result, the concentration of the electrically conducting filler may be reduced and the elasticity of the dielectric elastomer switch may be enhanced resulting in a softer sensor system.

If the pressure exerted by the stimuli-responsive elastomer is released, the shape of the piezoresistive elastomeric composite structure is not only restored by the elastic force of the piezoresistive elastomeric composite structure but may also be restored by the elastic force of the electrically insulating elastomer in which the piezoresistive elastomeric composite structure is embedded. This enables a very fast and complete recovery of the piezoresistive elastomeric composite structure to its initial state. Hence a soft sensor system with a high repeatability and response time for the measurement of external stimuli may be provided.

The elastic force of the piezoresistive elastomeric composite structure and the electrically insulating elastomer also acts as a counter force to the pressure which is applied to the dielectric elastomer switch leading to a compression or expansion of the piezoresistive elastomeric composite structure. Therefore, the degree of compression or expansion and accordingly the sensitivity of the sensor system may be adjusted by the elasticity of the piezoresistive elastomeric composite structure and the electrically insulating elastomer.

The electrically insulating elastomer may be made of the same material as the dielectric elastomeric matrix of the piezoelectric elastomeric composite structure. If the electrically insulating elastomer and the dielectric elastomeric matrix of the piezoelectric elastomeric composite are produced from different materials, it is favourable that the materials are chosen so that the Young's modulus of the electrically insulating elastomer, in which the piezoelectric elastomeric composite is embedded, may be lower than or equal to the Young's modulus of the piezoelectric elastomeric composite. A lower or equal Young's Modulus ensures that insulating polymer material does not hinder the expansion of the stimuli-responsive polymer and, thus, ensures a sufficient compression of the piezoresistiven dielectric elastomer switch.

The dielectric elastomer switch can be compressed or expanded by the pressure exerted by the stimuli-responsive elastomer. The pressure can be induced by a change in the volume, i. e. an increase or a decrease of the volume of the stimuli-responsive elastomer in response to an externally applied chemical or physical stimulus interacting with the stimuli-responsive elastomer. In the sensor system, the stimuli-responsive elastomer is preferably a chemo-responsive elastomer configured to exhibit a change in volume that exerts pressure on the contact surface in response to a change in a concentration of a chemical and/or a change in a redox potential and/or a change in a pH-level applied to the stimuli-responsive elastomer, a thermo-responsive elastomer configured to exhibit a change in volume that exerts pressure on the contact surface in response to a change in the temperature of the stimuli-responsive elastomer and/or a radiation-responsive or photo-responsive elastomer configured to exhibit a change in volume that exerts pressure on the contact surface in response to a change in the wavelength or intensity of an electromagnetic wave incident on the stimuli-responsive elastomer.

In this application, chemo-responsive elastomers are meant to also include bio-responsive elastomers. These elastomers are stimuli-responsive elastomers which exhibit a change of their volume in response to a biological stimulus, for example an enzyme, a hormone like insulin, or glucose. Besides, the stimuli-responsive elastomer may be a dual stimuli-responsive elastomer that simultaneously responds to two external stimuli or a multiple stimuli-responsive elastomer that simultaneously responds to more than one external stimulus.

Depending on the nature of the stimuli-responsive elastomer the volume of the stimuli-responsive elastomer may increase, i. e. expand, up to a maximum volume or decrease, i. e. shrink, to a minimum volume when an external stimulus interacts with the stimuli-responsive elastomer. During the volume change the stimuli-responsive elastomer preferably maintains its overall three-dimensional shape. If, for example, the stimuli-responsive elastomer is a stimuli-responsive hydrogel, it may in crease in volume by swelling due to absorption of a specific solvent that acts as an external stimulus when the stimuli-responsive hydrogel is brought into contact with the solvent. If, on the other hand, a thermo-responsive polymer is used as stimuli-responsive elastomer, this stimuli-responsive elastomer may decrease in volume when it is exposed to an elevated temperature above room temperature as an external stimulus.

Since stimuli-responsive elastomers can exhibit an increase or decrease in volume when an external stimulus interacts with them, different switching functions can be realized with the sensor system depending on the volume change and the initial state of the stimuli-responsive elastomer. The initial state is the state of the stimuli-responsive elastomer before or at the beginning of a measurement or switching. For example, a normally open switching function may be realized if the electric resistivity of the dielectric elastomer switch decreases under compression or expansion and the stimuli-responsive elastomer exhibit its original intrinsic volume in the initial state in the absence of an external stimulus. A normally closed switching function may be realized by applying an initial compression or tension to the dielectric elastomer switch in the initial state, for example by fixing the stimuli-responsive in the presence of an external stimulus in an expanded or shrunk initial state. Furthermore, the sensor system may be set up to detect or switch at a certain threshold of the external stimulus.

If a stimuli-responsive elastomer that expands when it interacts with an external stimulus is fixed relative to the dielectric elastomer switch at a distance smaller than the maximum expansion of the stimuli-responsive elastomer while the stimuli-responsive elastomer exhibits its original volume in the initial state, the external stimulus will not be detected until the external stimulus reaches a threshold which is large enough to expand the stimuli-responsive elastomer to such an extent that the stimuli-responsive elastomer bridges the distance between the stimuli-responsive elastomer and the dielectric elastomer switch and contacts the dielectric elastomer switch at the contact surface. For a fast response of the sensing system the contact between the stimuli-responsive elastomer and the dielectric elastomer switch at the contact surface may be established by a direct contact of the stimuli-responsive elastomer to the dielectric elastomer switch at the contact surface. Nevertheless, the contact may also be established by an intermediate element or intermediate layer which transfers the pressure exerted by the stimuli-responsive elastomer to the contact surface at the dielectric elastomer switch. A switching threshold can also be realized by defining the Young's modulus of the insulating polymer. The stiffer the insulating polymer is, the more pressure has to be applied by the stimuli-responsive polymer in order to trigger the switch. Furthermore, several dielectric elastomer switches with different loads of conductive fillers can be integrated. Different filler loads lead to different amounts of compression or elongation and, thus, amount of swelling and a value of stimuli that is needed to trigger the individual dielectric elastomer switch. This leads to a mulit-point-of-care sensor.

The dielectric elastomer switch comprises at least one cavity or opening in which the stimuli-responsive elastomer is embedded. By embedding the stimuli-responsive elastomer in the cavity or opening the stimuli-responsive elastomer can be easily fixed relative to the dielectric elastomer switch and a large ratio of contact surface to volume of the stimuli-responsive elastomer can be provided for the sensing. Most preferably, the stimuli-responsive elastomer and the dielectric elastomer switch may be rotationally symmetric and the contact surface may be constituted at the circumferential surface of the stimuli-responsive elastomer and the circumferential surface of the cavity or the opening in the dielectric elastomer switch. If the pressure on the dielectric elastomer switch is exerted by the stimuli-responsive elastomer via a circumferential surface of the stimuli-responsive elastomer, irregularities in the volume change of dielectric elastomer switch may be compensated and a high reproducibility of the measurements may be attained with the sensor system.

In one embodiment, the dielectric elastomer switch and the stimuli-responsive elastomer of the sensor system are embedded in an additional polymer layer and a polymer shell which are configured to be permeable for the external stimulus. The polymer layer and the polymer shell are configured to exert a pressure on a surface of the dielectric elastomer switch which opposes the contact surface to the stimuli-responsive elastomer whereby the pressure counteracts the pressure exerted by the stimuli-responsive elastomer. A pressure exerted by the polymer layer and the polymer shell on the dielectric elastomer is regarded as a counteracting pressure if the normal force that is equivalent to the pressure is directed in an opposing direction to the normal force which is equivalent to the pressure exerted by the stimuli-responsive elastomer on the dielectric elastomer switch. Due to the opposing forces a stronger deformation, i. e. compression or expansion, of the dielectric elastomer switch can be achieved during the volume change of the stimuli-responsive elastomer. As a result, the change in the electric output signal of dielectric elastomer switch may be enhanced and the signal-to-noise ratio may be improved significantly. If the polymer layer's Young's modulus is significantly higher than the Young's modulus of the stimuli-responsive polymer and the dielectric elastomer switch (i.e., typically at least 10 percent higher), it reduces the amount of perpendicular swelling and, thus, forces the swelling polymer to expand in the direction of the dielectric elastomer switch. The directed swelling will mainly compress the insulating polymer and the switch and, thus, will increase the sensitivity of the entire system.

The counteracting pressure or force may in particular be achieved in a sensor system wherein the polymer layer and the polymer shells are elastomers and the polymer layer exhibits a lower Young's modulus than the Young's modulus of the polymer shell. In the sensor system any surfaces of the dielectric elastomer switch and the stimuli-responsive elastomer which are directed in different spatial directions compared to the contact surface can be embedded in the polymer layer. The polymer layer and the surface of the dielectric elastomer switch which opposes the contact surface can be embedded in the polymer shell. When the stimuli-responsive elastomer expands, the elastic polymer layer pushes the polymer shell outwards at the surfaces which are directed in different spatial directions compared to the contact surface. This leads to a constriction of the stiffer polymer shell so that the polymer shell is pulled towards the dielectric elastomer switch at the surface of the dielectric elastomer switch which opposes the contact surface to the stimuli-responsive elastomer and thus counteracts the pressure of the expanding stimuli-responsive elastomer. In a sensing system with a shrinking stimuli-responsive elastomer the polymer layer and the polymer shell will have an analogous effect. Since the effect may already be attained with a thin polymer layer and polymer shell of typically 50 µm to 5 mm, the amount of stimuli-responsive elastomer and thus the overall size of the sensor system may be significantly reduced so that the system may be easily integrated into miniaturized soft robotic structures, for example into an automated insulin pump.

The sensor system may also be used in soft digital logic elements. A digital logic element may comprise at least one sensor system as described wherein the at least one sensor system is configured as a Boolean logic gate that performs a Boolean logical operation on the electric output signal of the at least one sensor system and produces a binary electric output signal depending on the logical operation. The basic Boolean logical operations are the NOT, AND, NAND, OR, NOR, XOR and XNOR operation. However, the digital logic element may also be used as a building block for more complex soft logic circuit components or soft logic circuits enabling information processing.

The sensor system and the digital logic elements may further be used to supply soft robotic systems with the necessary control signals for an autonomous, meaning self-controlled operation. A robotic system comprises at least one sensor system as described and/or at least one digital logic element as described, an actuator and a control unit. The control unit is configured to control the actuator in dependence of the electric output signal of the at least one sensor system and/or the electric output signal of the at least on digital logic element. Since the sensor system and/or the digital logic element and the actuator can be built from entirely soft materials, a robotic system with an increased flexibility and adaptability for accomplishing tasks, as well as an improved safety when working around humans may be provided.

In a method for determining an external stimulus a pressure is exerted on a contact surface of a dielectric elastomer switch by a stimuli-responsive elastomer which contacts the dielectric elastomer switch at the contact surface and exerts the pressure on the contact surface in response to an external stimulus that interacts with the stimuli-responsive elastomer. The pressure exerted on the contact surface is detected by a dielectric elastomer switch that generates an electric output signal in dependence of the detected pressure.

The described method can be carried out in particular with the sensor system or the digital logic element described, that is, the sensor system and the digital logic element described are set up to carry out the described method.

Exemplary embodiments of the invention are illustrated in the drawings and will be explained below with reference to figures 1 to 4.

In the figures:
- Fig. 1: shows a schematic cross-sectional oblique view of a stimuli-responsive sensor system,
- Fig. 2: shows a schematic cross-sectional top view of a stimuli-responsive sensor system without an external stimulus and if an external stimulus is applied,
- Fig. 3: shows a schematic cross-sectional side view of a stimuli-responsive sensor system without an external stimulus and if an external stimulus is applied, and
- Fig. 4: shows a schematic cross-sectional top view of a digital logic element.

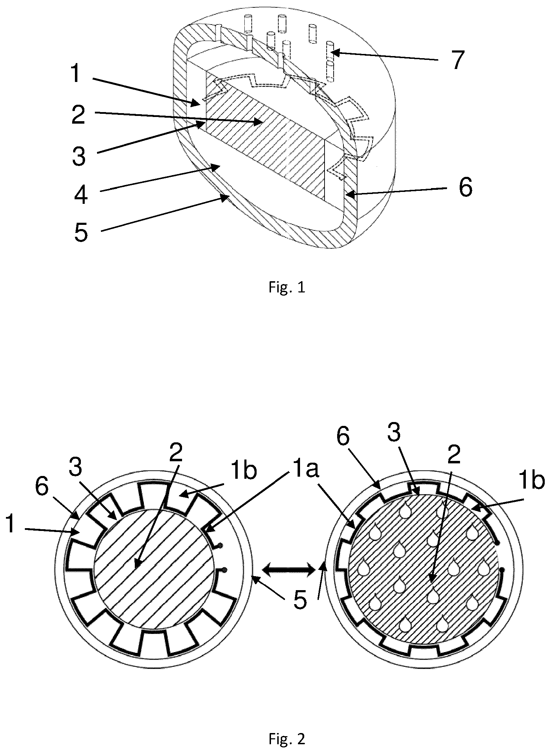

Figure 1 depicts a schematic cross-sectional oblique view of a stimuli-responsive sensor system. The sensor system comprises a dielectric elastomer switch 1 and a stimuli-responsive elastomer 2. The dielectric elastomer switch 1 and the stimuli-responsive elastomer 2 are configured to contact each other at a contact surface 3. The stimuli-responsive elastomer 2 is further configured to exert a pressure on this contact surface 3 if an external stimulus interacts with the stimuli-responsive elastomer 2. The dielectric elastomer switch 1 is configured to detect the pressure exerted on the contact surface 3 by the stimuli-responsive elastomer 2 and to generate an electric output signal in dependence of the detected pressure.

The dielectric elastomer switch 1 is constructed with a piezoresistive elastomeric composite structure 1a, shown in figure 2, comprising an electrically conducting filler, in the example carbon black powder, that is dispersed in a dielectric elastomeric matrix, in the example in silicone or silicone grease. The piezoresistive elastomeric composite structure 1a may be embedded in an electrically insulating elastomer 1b, shown in figure 2, such as rubber or an elastomeric organosilicon compound. The piezoresistive elastomeric composite structure 1a is further configured to exhibit an elastic deformation that changes the electrical resistivity of the piezoresistive elastomeric composite structure 1a when a pressure is exerted on the piezoresistive elastomeric composite structure 1a by the stimuli-responsive elastomer 2 via the contact surface 3 and the electrically insulating elastomer 1b, respectively. In its initial undeformed state the piezoresistive elastomeric composite structure 1a possesses an electrical resistance of R > 10⁹ Ω. If the piezoresistive elastomeric composite structure 1a is deformed by the pressure exerted by the stimuli-responsive elastomer 2 the resistivity can decrease by at least five orders of magnitude.

In the example of figure 1 the piezoresistive elastomeric composite structure 1a is a planar structure comprising multiple elongated sections which are oriented with their elongated axis parallel to the direction of the pressure action of the stimuli-responsive elastomer 2, i. e. perpendicular to the contact surface 3 between the dielectric elastomer switch 1 and the stimuli-responsive elastomer 2. The elongated sections are connected in series forming a square wave structure. The reversible change in resistivity of this piezoresistive elastomeric composite structure 1a therefore stems from a percolation effect of the electrically conducting filler which forms conductive pathways when the piezoresistive elastomeric composite structure 1a is deformed and from a change in the geometry of the piezoresistive elastomeric composite structure 1a during its deformation. The electrically insulating elastomer 1b acts as a structural support to the piezoresistive elastomeric composite structure 1a and aids the restoring of the shape of the piezoresistive elastomeric composite structure 1a. Besides this, the electrically insulating elastomer 1b protects the piezoresistive elastomeric composite structure 1a from overpressure and also from the external stimulus. This is particularly advantageous in sensor systems with electrically conducting fillers which may degrade if the external stimulus interacts with the electrically conducting filler, e. g. by oxidizing a metallic electrically conducting filler.

The stimuli-responsive elastomer 2 in the example of figure 1 is a chemo-responsive elastomer which exerts a pressure on the dielectric switch 1 by a change in volume when the stimuli-responsive elastomer 2 interacts with a chemical as an external stimulus, for example a solvent which is absorbed by the stimuli-responsive elastomer 2 resulting in a swelling of the stimuli-responsive elastomer 2. Alternatively to a volume change by an increase in the volume, the sensor system may comprise a stimuli-responsive elastomer 2 that exhibits a decrease in volume when it interacts with an external stimulus. The external stimulus may be a chemical and/or biological stimulus, like a change in a redox potential, pH-level, glucose level, enzyme level or hormone level, and/or a physical stimulus like a change in temperature and/or a change in the wavelength or intensity of an incident electromagnetic wave.

In order to exert a pressure on the dielectric elastomer switch 1, the stimuli-responsive elastomer 2 is fixed relatively to the dielectric elastomer switch 1. According to the invention, fig. 1 shows the stimuli-responsive elastomer 2 embedded in a cavity or opening in the elastomer switch 1 to fix the stimuli-responsive elastomer 2 relative to the dielectric elastomer switch 1. For a uniform deformation of the dielectric elastomer switch 1, the stimuli-responsive elastomer 2 and the cavity or opening in the dielectric elastomer switch 1 may be rotationally symmetric shaped and the contact surface 3 may be a circumferential surface of the stimuli-responsive elastomer 2 and a circumferential surface of the cavity or opening in the elastomer switch 1 perpendicular to the axis of rotation. However, other shapes of the stimuli-responsive elastomer 2 and the cavity or opening in the elastomer switch 1, for example elongated bar like shapes, may also be viable.

In the example of figure 1, the stimuli-responsive elastomer 2, the dielectric elastomer switch 1 and the cavity or opening in the dielectric elastomer switch 1 are cylindrically shaped and the contact surface 3 is constituted by the lateral surface of the stimuli-responsive elastomer 2 and the lateral surface of the cavity or opening in the elastomer switch 1. The stimuli-responsive elastomer 2 and the elastomer switch 1 are in direct contact to each other at the contact surface 3 in the example, meaning the stimuli-responsive elastomer 2 and the elastomer switch 1 touch each other. However, the contact between the stimuli-responsive elastomer 2 and the elastomer switch 1 may also be established by an intermediate element or intermediate layer which can be positioned between the stimuli-responsive elastomer 2 and the elastomer switch 1 to transfer the pressure exerted by the stimuli-responsive elastomer 2 from the stimuli-responsive elastomer 2 to the contact surface 3 at the dielectric elastomer switch 1.

The sensor system may, based on the same working principle, exhibit different measuring or switching functions depending on the initial state and the volume change of the stimuli-responsive elastomer 2. In the example of figure 1, the sensor system may be set up as normally open switch. The stimuli-responsive elastomer 2 may exhibit its original, intrinsic volume in the initial state, i. e. before or at the beginning of a measurement. When an external stimulus interacts with the stimuli-responsive elastomer 2, the volume of the stimuli-responsive elastomer 2 changes and a pressure is exerted on the dielectric elastomer switch 1. The dielectric elastomer switch 1 is deformed by the pressure and thus the electric resistivity of the dielectric elastomer switch 1 decreases. Alternatively, the switch 1 may be set up as a normally closed switch if the stimuli-responsive elastomer 2 exhibits an increased or decreased volume in its initial state. Analogous switching functions may be achieved with a stimuli-responsive elastomer 2 that exhibits a decrease in its volume when it interacts with an external stimulus and a dielectric elastomer switch 1 which exhibits an increase in its resistivity when deformed. Furthermore, a threshold level for the measurement or switching may be set by a gap between the dielectric elastomer switch 1 and stimuli-responsive elastomer 2 which is bridged by the expanding stimuli-responsive elastomer 2 when the external stimulus reaches the threshold level.

For an enhanced signal, the sensor system in the example of figure 1 further comprises an additional polymer layer 4 and a polymer shell 5 which are permeable for the external stimulus and which are configured to exert a pressure on a surface 6 of the dielectric elastomer switch 1 which opposes the contact surface 3 whereby the pressure counteracts the pressure exerted by the stimuli-responsive elastomer 2. The polymer layer 4 and the polymer shell 5 may, for example, be configured with through holes 7, as shown in figure 1, so that an external chemical stimulus can pass through the holes 7 in order to interact with the stimuli-responsive elastomer 2. Any surfaces of the dielectric elastomer switch 1 and the stimuli-responsive elastomer 2 which are directed in different spatial directions compared to the contact surface 3 may be embedded in or covered by the polymer layer 4 which exhibits a lower Young's modulus than the Young's modulus of the polymer shell 5. The entire outer surface of the polymer layer 4 and the surface 6 of the dielectric elastomer switch 1 which opposes the contact surface 3 may be embedded in the polymer shell 5. When the stimuli-responsive elastomer 2 changes its volume, the stiffer polymer shell 5 will be constricted at the surface 6 when the stimuli-responsive elastomer 2 expands, or bulge outwards at the surface 6 when the stimuli-responsive elastomer 2 shrinks so that it counteracts the pressure of the stimuli-responsive elastomer 2. Due to this counteracting pressure a stronger compression or expansion of the dielectric elastomer switch 1 may be achieved and the electric output signal of the sensor system may be enhanced significantly.

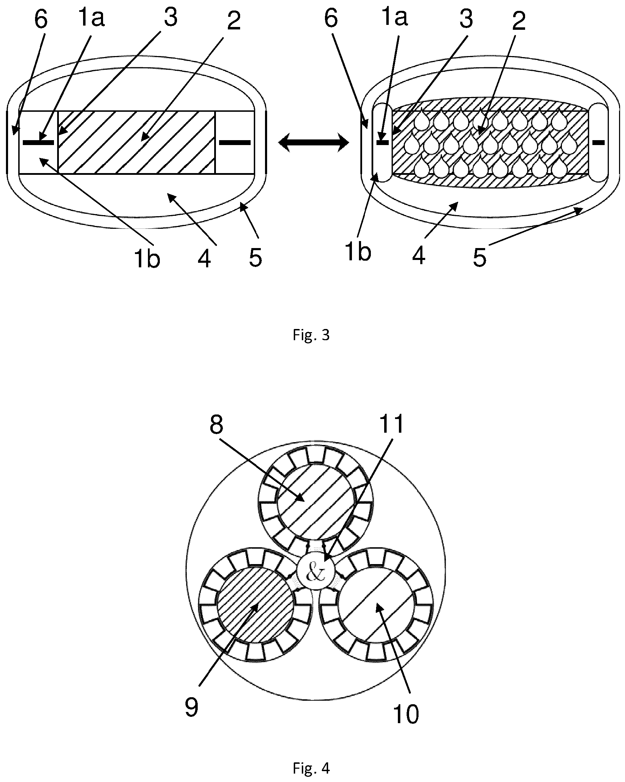

Figure 2 shows the sensor system of figure 1 in a schematic cross-sectional top view and figure 3 shows the sensor system of figure 1 in a schematic cross-sectional side view. Recurring features in these figures and in the following figure are in each case denoted with identical reference signs. On the left hand side of figure 2 and figure 3 the sensor system is depicted if no external stimulus interacts with the stimuli-responsive elastomer 2. On the right hand side of figure 2 and figure 3 the sensor system is depicted if an external stimulus interacts with the stimuli-responsive elastomer 2. If the external stimulus does not interact with the stimuli-responsive elastomer 2, the stimuli-responsive elastomer 2 exhibits its original intrinsic volume and the piezoresistive elastomeric composite structure 1a of the dielectric elastomer switch 1 does not experience any compression or tension and is thus undeformed. If an external stimulus interacts with the stimuli-responsive elastomer 2, the stimuli-responsive elastomer 2 experiences a reversible change in volume, in the example of the figures 2 and 3 an in crease in volume. This increase in the volume leads to an expansion of the stimuli-responsive elastomer 2 so that a pressure is exerted by the stimuli-responsive elastomer 2 on the dielectric elastomer switch 1 which is compressed by this pressure.

In the example of figures 2 and 3 the stimuli-responsive elastomer 2 is cylindrically shaped and expands radially. The piezoresistive elastomeric composite structure 1a of the elastomer switch 1 comprises multiple elongated sections which are oriented with their elongated axis in the direction of the pressure action of the stimuli-responsive elastomer 2. In the example of figure 2 and 3, these sections are oriented in a radial direction with respect to the cylindrically shaped stimuli-responsive elastomer 2 so that they experience a strong compression in the direction of their longitudinal axis when the dielectric elastomer switch 1 and therefore the electrically insulating elastomer 1b and the piezoresistive elastomeric composite structure 1a are compressed. The compression causes a change in the resistivity of the piezoresistive elastomeric composite structure 1a which is detected and used to generate an electric output signal of the sensor system. The compression and thus the electric output signal of the sensor system may be enhanced by a polymer layer 4 and a polymer shell 5 which are configured to exert a pressure on the dielectric elastomer switch 1 that counteracts the pressure of the stimuli-responsive elastomer 2 at a surface 6 opposite to the contact surface 3. Analogous solutions may be attained with stimuli-responsive elastomers 2 which exhibit a decrease in volume resulting in a stretching of the dielectric elastomer switch 1.

Figure 4 illustrates a digital logic element in a schematic cross-sectional top-view. The digital logic element comprises three sensor systems 8, 9, 10 which are configured as a Boolean logic gate 11 that performs a Boolean logical operation on the electric output signal of the sensor systems 8, 9, 10 and produces a binary electric output signal depending on this logical operation. In the example of figure 4 the Boolean logic gate 11 performs an AND operation. The Boolean logic gate may, however also perform other operations such as NOT, NAND, OR, NOR, XOR and XNOR operations. The logic sensor array may be embedded in the same material as is used for the polymer layer 4. The logic gates that evaluate the sensor information may consist of digital dielectric elastomer switch units, comprising a dielectric elastomer actuator and a dielectric elastomer switch as described above. All electrically conductive materials such as conductive connection, electrical resistors, dielectric elastomer actuators and switches that may be part of the logic circuit may comprise a conductive filter dispersed in a polymer matrix and can be seen as flexible structures.

The digital logic element may be used as a building block for more complex soft logic circuit components or soft logic circuits enabling information processing. The sensor system and/or the digital logic element may particularly also be used to supply robotic systems with the necessary control signals for an autonomous operation. Such a robotic system comprises at least one sensor system and/or at least one digital logic element, an actuator and a control unit which is configured to control the actuator in dependence of the output signal of the at least one sensor system and/or the output signal of the at least one digital logic element. Since the actuator and the sensor system and/or the digital logic element may be built from entirely soft materials a robotic system with a high flexibility, compliance and sensitivity and thus improved safety may be provided which may be especially suitable for medical applications and collaborative robotics.

The project leading to this application has received funding from the European Union's Horizon 2020 research and innovation programme under the Marie Sklodowska-Curie grant Agreement No 706754.

## Claims

1. Sensor system comprising a dielectric elastomer switch (1) and a stimuli-responsive elastomer (2), wherein
the dielectric elastomer switch (1) comprises a piezoresistive elastomeric composite structure (1a) that is embedded in an electrically insulating elastomer (1b), and the stimuli-responsive elastomer (2) are configured to contact each other at a contact surface (3),
the stimuli-responsive elastomer component (2) is configured to exert a pressure on the contact surface (3) if an external stimulus interacts with the stimuli-responsive elastomer (2) and causes a reversible change in volume of the stimuli-responsive elastomer (3) in response to the external stimulus and
the dielectric elastomer switch (1) is configured to detect the pressure exerted on the contact surface (3) by the stimuli-responsive elastomer (2) and to generate an electric output signal in dependence of the detected pressure, and the stimuli-responsive elastomer (2) is
a chemo-responsive elastomer configured to exhibit a change in volume that exerts pressure on the contact surface (3) in response to a change in a concentration of a chemical and/or a change in a redox potential and/or a change in a pH-level applied to the stimuli-responsive elastomer (2),
a thermo-responsive elastomer configured to exhibit a change in volume that exerts pressure on the contact surface (3) in response to a change in the temperature of the stimuli-responsive elastomer (2),
a radiation-responsive elastomer configured to exhibit a change in volume that exerts pressure on the contact surface (3) in response to a change in the wavelength or intensity of an electromagnetic wave incident on the stimuli-responsive elastomer (2) and/or
a bio-responsive elastomer configured to exhibit a change in volume that exerts pressure on the contact surface (3) in response to a biological stimulus, **characterized in that**
the dielectric elastomer switch (1) comprises at least one cavity or opening in which the stimuli-responsive elastomer (2) is embedded.

2. Sensor system according to claim 1, **characterized in that**
the piezoresistive elastomeric composite structure (1a) comprises an electrically conducting filler dispersed in a dielectric elastomeric matrix and
the piezoresistive elastomeric composite structure (1a) is configured to exhibit an elastic deformation that alters the electric resistivity of the piezoresistive elastomeric composite structure (1a) when a pressure is exerted on the electrically insulating elastomer (1b) and the piezoresistive elastomeric composite structure (1a) by the stimuli-responsive elastomer (2) via the contact surface (3).

3. Sensor system according to claim 1, **characterized in that** the contact surface (3) is constituted at a circumferential surface of the stimuli-responsive elastomer (2) and a circumferential surface of the cavity or the opening in the dielectric elastomer switch (1).

4. Sensor system according to one of the preceding claims, **characterized in that**
the dielectric elastomer switch (1) and the stimuli-responsive elastomer (2) are embedded in a polymer layer (4) and a polymer shell (5) wherein
the polymer layer (4) and the polymer shell (5) are configured to exert a pressure on a surface (6) of the dielectric elastomer switch (1) which opposes the contact surface (3) to the stimuli-responsive elastomer (2) whereby the pressure counteracts the pressure exerted by the stimuli-responsive elastomer (2) at the contact surface (3) and
the polymer layer (4) and the polymer shell (5) are configured to be permeable for the external stimulus.

5. Sensor system according to claim 4, **characterized in that**
any surfaces of the dielectric elastomer switch (1) and the stimuli-responsive elastomer (2) that are directed in different spatial directions compared to the contact surface (3) are embedded in the polymer layer (4),
the outer surface of the polymer layer (4) and any surfaces of the dielectric elastomer switch (1) which oppose the contact surface (3) are embedded in the polymer shell (5) and
the polymer layer (4) and the polymer shell (5) are elastomers wherein the polymer layer (4) exhibits a lower Young's modulus than the Young's modulus of the polymer shell (5).

6. Digital logic element comprising at least one sensor system (8, 9, 10) according to one of the preceding claims, **characterized in that** the at least one sensor system is configured as a Boolean logic gate (11) that performs a Boolean logical operation on the electric output signal of the at least one sensor system and produces a binary electric output signal depending on the logical operation.

7. Robotic system comprising at least one sensor system according to any of claims 1 to 5 or at least one digital logic element according to claim 6, a control unit and an actuator **characterized in that** the control unit is configured to control the actuator in dependence of the electric output signal of the at least one sensor system or the electric output signal of the at least one digital logic element.

8. Method for determining an external stimulus by
exerting a pressure on a contact surface (3) of a dielectric elastomer switch (1) by a reversible change in volume of a stimuli-responsive elastomer (2) which contacts the dielectric elastomer switch (1) at the contact surface (3) and exerts the pressure on the contact surface (3) in response to an external stimulus interacting with the stimuli-responsive elastomer (2), wherein the dielectric elastomer switch (1) comprises a piezoresistive elastomeric composite structure (1a) that is embedded in an electrically insulating elastomer (1b),
detecting the pressure exerted onto the contact surface (3) by the dielectric elastomer switch (1) and
generating an electric output signal by the dielectric elastomer switch (1) in dependence of the detected pressure and the stimuli-responsive elastomer (2) is
a chemo-responsive elastomer configured to exhibit a change in volume that exerts pressure on the contact surface (3) in response to a change in a concentration of a chemical and/or a change in a redox potential and/or a change in a pH-level applied to the stimuli-responsive elastomer (2),
a thermo-responsive elastomer configured to exhibit a change in volume that exerts pressure on the contact surface (3) in response to a change in the temperature of the stimuli-responsive elastomer (2),
a radiation-responsive elastomer configured to exhibit a change in volume that exerts pressure on the contact surface (3) in response to a change in the wavelength or intensity of an electromagnetic wave incident on the stimuli-responsive elastomer (2) and/or
a bio-responsive elastomer configured to exhibit a change in volume that exerts pressure on the contact surface (3) in response to a biological stimulus, **characterized in that**
the dielectric elastomer switch (1) comprises at least one cavity or opening in which the stimuli-responsive elastomer (2) is embedded.

## Patentansprüche

1. Sensorsystem, das einen dielektrischen Elastomerschalter (1) und ein reizempfindliches Elastomer (2) umfasst, wobei
der dielektrische Elastomerschalter (1) eine piezoresistive Elastomer-Verbundstruktur (1a) umfasst, die in ein elektrisch isolierendes Elastomer (1b) eingebettet ist, und das reizempfindliche Elastomer (2) eingerichtet ist, an einer Kontaktfläche (3) mit dem piezoresistiven Elastomer in Kontakt zu stehen,
die reizempfindliche Elastomerkomponente (2) eingerichtet ist, einen Druck auf die Kontaktfläche (3) auszuüben, wenn ein äußerer Reiz mit dem reizempfindlichen Elastomer (2) in Wechselwirkung tritt und eine reversible Volumenänderung des reizempfindlichen Elastomers (3) als Reaktion auf den äußeren Reiz bewirkt, und
der dielektrische Elastomerschalter (1) eingerichtet ist, den auf die Kontaktfläche (3) durch das reizempfindliche Elastomer (2) ausgeübten Druck zu erfassen und in Abhängigkeit vom erfassten Druck ein elektrisches Ausgangssignal zu erzeugen, und das reizempfindliche Elastomer (2) ist
ein chemisch reaktives Elastomer, das eingerichet ist, eine Volumenänderung aufzuweisen, die einen Druck auf die Kontaktfläche (3) ausübt als Reaktion auf eine Änderung der Konzentration einer Chemikalie und/oder eine Änderung des Redoxpotentials und/oder eine Änderung des pH-Werts, die auf das reizempfindliche Elastomer (2) ausgeübt werden,
ein thermoresponsives Elastomer, das eingerichtet ist, eine Volumenänderung aufzuweisen, die als Reaktion auf eine Temperaturänderung des reizempfindlichen Elastomers (2) Druck auf die Kontaktfläche (3) ausübt,
ein strahlungsempfindliches Elastomer, das eingerichtet ist, eine Volumenänderung aufzuweisen, die einen Druck auf die Kontaktfläche (3) ausübt als Reaktion auf eine Änderung der Wellenlänge oder Intensität einer elektromagnetischen Welle, die auf das reizempfindliche Elastomer (2) auftrifft, und/oder
ein bio-responsives Elastomer, das eingerichtet ist, eine Volumenänderung aufzuweisen, die als Reaktion auf einen biologischen Reiz Druck auf die Kontaktfläche (3) ausübt, **dadurch gekennzeichnet, dass**
der dielektrische Elastomerschalter (1) mindestens einen Hohlraum oder eine Öffnung umfasst, in den bzw. die das reizempfindliche Elastomer (2) eingebettet ist.

2. Sensorsystem nach Anspruch 1, **dadurch gekennzeichnet, dass**
die piezoresistive elastomere Verbundstruktur (1a) einen elektrisch leitenden Füllstoff aufweist, der in einer dielektrischen elastomeren Matrix dispergiert ist, und
die piezoresistive elastomere Verbundstruktur (1a) eingerichtet ist, eine elastische Verformung aufzuweisen, die den elektrischen Widerstand der piezoresistiven elastomeren Verbundstruktur (1a) verändert, wenn durch das reizempfindliche Elastomer (2) ein Druck auf das elektrisch isolierende Elastomer (1b) und die piezoresistive elastomere Verbundstruktur (1a) über die Kontaktfläche (3) ausgeübt wird.

3. Sensorsystem nach Anspruch 1, **dadurch gekennzeichnet, dass** die Kontaktfläche (3) an einer Umfangsfläche des reizempfindlichen Elastomers (2) und einer Umfangsfläche des Hohlraums oder der Öffnung im dielektrischen Elastomerschalter (1) ausgebildet ist.

4. Sensorsystem nach einem der vorhergehenden Ansprüche, **dadurch gekennzeichnet, dass**
der dielektrische Elastomerschalter (1) und das reizempfindliche Elastomer (2) in eine Polymerschicht (4) und eine Polymerschale (5) eingebettet sind, wobei
die Polymerschicht (4) und die Polymerschale (5) eingerichtet sind, einen Druck auf die Oberfläche (6) des dielectrischen Elastomerschalters (1) auszuüben, die der Kontaktfläche (3) gegenüber dem reizempfindlichen Elastmoter (2) liegt, wodurch der Druck dem von dem reizempfindlichen Elastometer (2) an der Kontaktfläche (3) ausgeübten Druck entgegenwirkt, und
die Polymerschicht (4) und die Polymerschale (5) eingerichtet sind, für den äußeren Reiz durchlässig zu sein.

5. Sensorsystem nach Anspruch 4, **dadurch gekennzeichnet, dass**
alle Oberflächen des dielektrischen Elastomerschalters (1) und des reizempfindlichen Elastomers (2), die im Vergleich zur Kontaktfläche (3) in unterschiedliche räumliche Richtungen weisen, in die Polymerschicht (4) eingebettet sind,
die Außenfläche der Polymerschicht (4) und alle Flächen des dielektrischen Elastomerschalters (1), die der Kontaktfläche (3) gegenüberliegen, in die Polymerschale (5) eingebettet sind und
die Polymerschicht (4) und die Polymerschale (5) Elastomere sind, wobei die Polymerschicht (4) einen geringeren Elastizitätsmodul als die Polymerschale (5) aufweist.

6. Digitales Logikelement, das mindestens ein Sensorsystem (8, 9, 10) gemäß einem der vorhergehenden Ansprüche umfasst, **dadurch gekennzeichnet, dass** das mindestens eine Sensorsystem als Boolesches Logikgatter (11) eingerichtet ist, eine Boolesche Logikoperation an dem elektrischen Ausgangssignal des mindestens einen Sensorsystems durchführt und in Abhängigkeit von der Logikoperation ein binäres elektrisches Ausgangssignal erzeugt.

7. Robotersystem, umfassend mindestens ein Sensorsystem gemäß einem der Ansprüche 1 bis 5 oder mindestens ein digitales Logikelement gemäß Anspruch 6, eine Steuereinheit und einen Aktuator, **dadurch gekennzeichnet, dass** die Steuereinheit eingerichtet ist, den Aktuator in Abhängigkeit vom elektrischen Ausgangssignal des mindestens einen Sensorsystems oder vom elektrischen Ausgangssignal des mindestens einen digitalen Logikelements zu steuern.

8. Verfahren zum Bestimmen eines externen Reizes durch
Ausüben eines Drucks auf eine Kontaktfläche (3) eines dielektrischen Elastomerschalters (1) durch eine reversible Volumenänderung eines reizempfindlichen Elastomers (2), das den dielektrischen Elastomerschalter (1) an der Kontaktfläche (3) berührt und den Druck auf die Kontaktfläche (3) als Reaktion auf einen externen Reiz ausübt, der mit dem reizempfindlichen Elastomer (2) in Wechselwirkung steht, wobei der dielektrische Elastomerschalter (1) eine piezoresistive Elastomer-Verbundstruktur (1a) umfasst, die in ein elektrisch isolierendes Elastomer (1b) eingebettet ist,
Erfassen des Drucks, der durch den dielektrischen Elastomerschalter (1) auf die Kontaktfläche (3) ausgeübt wird, und
Erzeugen eines elektrischen Ausgangssignals durch den dielektrischen Elastomerschalter (1) in Abhängigkeit vom erfassten Druck und das reizempfindliche Elastomer (2) ist
ein chemisch reaktives Elastomer, das eingerichet ist, eine Volumenänderung aufzuweisen, die einen Druck auf die Kontaktfläche (3) ausübt als Reaktion auf eine Änderung der Konzentration einer Chemikalie und/oder eine Änderung des Redoxpotentials und/oder eine Änderung des pH-Werts, die auf das reizempfindliche Elastomer (2) ausgeübt werden,
ein thermoresponsives Elastomer, das eingerichtet ist, eine Volumenänderung aufzuweisen, die als Reaktion auf eine Temperaturänderung des reizempfindlichen Elastomers (2) Druck auf die Kontaktfläche (3) ausübt,
ein strahlungsempfindliches Elastomer, das eingerichtet ist, eine Volumenänderung aufzuweisen, die einen Druck auf die Kontaktfläche (3) ausübt als Reaktion auf eine Änderung der Wellenlänge oder Intensität einer elektromagnetischen Welle, die auf das reizempfindliche Elastomer (2) auftrifft, und/oder
ein bio-responsives Elastomer, das eingerichtet ist, eine Volumenänderung aufzuweisen, die als Reaktion auf einen biologischen Reiz Druck auf die Kontaktfläche (3) ausübt, **dadurch gekennzeichnet, dass**
der dielektrische Elastomerschalter (1) mindestens einen Hohlraum oder eine Öffnung umfasst, in den bzw. die das reizempfindliche Elastomer (2) eingebettet ist.

## Revendications

1. Système de capteur comprenant un commutateur en élastomère diélectrique (1) et un élastomère sensible aux stimuli (2), dans lequel
le commutateur en élastomère diélectrique (1), comprend une structure composite élastomère piézorésistive (1a) qui est intégrée dans un élastomère électriquement isolant (1b), et l'élastomère sensible aux stimuli (2) sont configurés pour venir en contact l'un avec l'autre au niveau d'une surface de contact (3),
le composant élastomère sensible aux stimuli (2) est configuré pour exercer une pression sur la surface de contact (3) si un stimulus externe interagit avec l'élastomère sensible aux stimuli (2) et provoque un changement réversible du volume de l'élastomère sensible aux stimuli (3) en réponse au stimulus externe et
le commutateur en élastomère diélectrique (1) est configuré pour détecter la pression exercée sur la surface de contact (3) par l'élastomère sensible aux stimuli (2) et pour générer un signal de sortie électrique en fonction de la pression détectée, et l'élastomère sensible aux stimuli (2) est
un élastomère sensible chimiquement configuré pour présenter un changement de volume qui exerce une pression sur la surface de contact (3) en réponse à un changement d'une concentration d'un produit chimique et/ou à un changement d'un potentiel d'oxydoréduction et/ou à un changement d'un niveau de pH appliqué à l'élastomère sensible aux stimuli (2),
un élastomère thermosensible configuré pour présenter un changement de volume qui exerce une pression sur la surface de contact (3) en réponse à un changement de la température de l'élastomère sensible aux stimuli (2),
un élastomère sensible à un rayonnement configuré pour présenter un changement de volume qui exerce une pression sur la surface de contact (3) en réponse à un changement de la longueur d'onde ou de l'intensité d'une onde électromagnétique incidente sur l'élastomère sensible aux stimuli (2) et/ou
un élastomère biosensible configuré pour présenter un changement de volume qui exerce une pression sur la surface de contact (3) en réponse à un stimulus biologique, **caractérisé en ce que**
le commutateur en élastomère diélectrique (1) comprend au moins une cavité ou ouverture dans laquelle l'élastomère sensible aux stimuli (2) est intégré.

2. Système de capteur selon la revendication 1, **caractérisé en ce que**
la structure composite élastomère piézorésistive (1a) comprend une charge électriquement conductrice dispersée dans une matrice élastomère diélectrique et
la structure composite élastomère piézorésistive (1a) est configurée pour présenter une déformation élastique qui modifie la résistivité électrique de la structure composite élastomère piézorésistive (1a) lorsqu'une pression est exercée sur l'élastomère électriquement isolant (1b) et la structure composite élastomère piézorésistive (1a) par l'élastomère sensible aux stimuli (2) via la surface de contact (3).

3. Système de capteur selon la revendication 1, **caractérisé en ce que** la surface de contact (3) est constituée au niveau d'une surface circonférentielle de l'élastomère sensible aux stimuli (2) et d'une surface circonférentielle de la cavité ou de l'ouverture dans le commutateur en élastomère diélectrique (1).

4. Système de capteur selon l'une quelconque des revendications précédentes, **caractérisé en ce que**
le commutateur en élastomère diélectrique (1) et l'élastomère sensible aux stimuli (2) sont intégrés dans une couche de polymère (4) et une enveloppe de polymère (5), dans lequel
la couche de polymère (4) et l'enveloppe de polymère (5) sont configurées pour exercer une pression sur une surface (6) du commutateur en élastomère diélectrique (1) qui oppose la surface de contact (3) à l'élastomère sensible aux stimuli (2), la pression s'opposant à la pression exercée par l'élastomère sensible aux stimuli (2) au niveau de la surface de contact (3) et
la couche de polymère (4) et l'enveloppe de polymère (5) sont configurées pour être perméables au stimulus externe.

5. Système de capteur selon la revendication 4, **caractérisé en ce que**
toutes les surfaces du commutateur en élastomère diélectrique (1) et de l'élastomère sensible aux stimuli (2) qui sont dirigées dans des directions spatiales différentes par rapport à la surface de contact (3) sont intégrées dans la couche de polymère (4),
la surface extérieure de la couche de polymère (4) et toutes les surfaces du commutateur en élastomère diélectrique (1) qui sont opposées à la surface de contact (3) sont intégrées dans l'enveloppe de polymère (5) et
la couche de polymère (4) et l'enveloppe de polymère (5) sont des élastomères, dans lequel la couche de polymère (4) présente un module de Young inférieur au module de Young de l'enveloppe de polymère (5).

6. Élément logique numérique comprenant au moins un système de capteur (8, 9, 10) selon l'une des revendications précédentes, **caractérisé en ce que** le au moins un système de capteur est configuré sous la forme d'une porte logique booléenne (11) qui effectue une opération logique booléenne sur le signal de sortie électrique du au moins un système de capteur et produit un signal de sortie électrique binaire en fonction de l'opération logique.

7. Système robotique comprenant au moins un système de capteur selon l'une quelconque des revendications 1 à 5 ou au moins un élément logique numérique selon la revendication 6, une unité de commande et un actionneur, **caractérisé en ce que** l'unité de commande est configurée pour commander l'actionneur en fonction du signal de sortie électrique du au moins un système de capteur ou du signal de sortie électrique du au moins un élément logique numérique.

8. Procédé pour déterminer un stimulus externe en
exerçant une pression sur une surface de contact (3) d'un commutateur en élastomère diélectrique (1) par un changement réversible de volume d'un élastomère sensible aux stimuli (2) qui vient en contact avec le commutateur en élastomère diélectrique (1) au niveau de la surface de contact (3) et exerce la pression sur la surface de contact (3) en réponse à un stimulus externe interagissant avec l'élastomère sensible aux stimuli (2), dans lequel le commutateur en élastomère diélectrique (1) comprend une structure composite élastomère piézorésistive (1a) qui est intégrée dans un élastomère électriquement isolant (1b),
détectant la pression exercée sur la surface de contact (3) par le commutateur en élastomère diélectrique (1) et
générant un signal de sortie électrique par l'intermédiaire du commutateur en élastomère diélectrique (1) en fonction de la pression détectée et l'élastomère sensible aux stimuli (2) est
un élastomère sensible chimiquement configuré pour présenter un changement de volume qui exerce une pression sur la surface de contact (3) en réponse à un changement d'une concentration d'un produit chimique et/ou à un changement d'un potentiel d'oxydoréduction et/ou à un changement d'un niveau de pH appliqué à l'élastomère sensible aux stimuli (2),
un élastomère thermosensible configuré pour présenter un changement de volume qui exerce une pression sur la surface de contact (3) en réponse à un changement de la température de l'élastomère sensible aux stimuli (2),
un élastomère sensible à un rayonnement configuré pour présenter un changement de volume qui exerce une pression sur la surface de contact (3) en réponse à un changement de la longueur d'onde ou de l'intensité d'une onde électromagnétique incidente sur l'élastomère sensible aux stimuli (2) et/ou
un élastomère biosensible configuré pour présenter un changement de volume qui exerce une pression sur la surface de contact (3) en réponse à un stimulus biologique, **caractérisé en ce que**
le commutateur en élastomère diélectrique (1) comprend au moins une cavité ou ouverture dans laquelle l'élastomère sensible aux stimuli (2) est intégré.
